# EUROPEAN PATENT SPECIFICATION

(11) **EP 2 818 546 B1**
(45) Date of publication and mention of the grant of the patent: **28.06.2017**
(21) Application number: 11875955.4
(22) Date of filing: 17.11.2011
(51) Int. Cl.: C12N 15/09, C12M 1/00, C12Q 1/68

(54) **METHOD FOR DETERMINING RHEUMATOID ARTHRITIS ACTIVITY INDICATOR, AND BIOMARKER USED THEREIN**
VERFAHREN ZUR BESTIMMUNG DES AKTIVITÄTSINDIKATORS VON RHEUMATOIDER ARTHRITIS UND BIOMARKER DAFÜR
PROCÉDÉ POUR LA DÉTERMINATION D'UN INDICATEUR DE L'ACTIVITÉ DE LA POLYARTHRITE RHUMATOÏDE, ET BIOMARQUEUR UTILISÉ DANS CE PROCÉDÉ

(43) Date of publication of application: 31.12.2014
(73) Proprietor: DNA Chip Research Inc., Yokohama-shi, Kanagawa 230-0045 (JP); Keio University, Tokyo 108-8345 (JP); Saitama Medical University, Iruma-gun, Saitama 350-0495 (JP)
(72) Inventor: TAKEUCHI, Tsutomu, Tokyo 160-8582 (JP); AMANO, Koichi, Iruma-gun Saitama 350-0495 (JP); ISHIZAWA, Yohei, Yokohama-shi Kanagawa 230-0045 (JP); NAKAMURA, Seiji, Yokohama-shi Kanagawa 230-0045 (JP); TANINO, Motohiko, Yokohama-shi Kanagawa 230-0045 (JP); HATA, Yuko, Yokohama-shi Kanagawa 230-0045 (JP)
(74) Representative: Dunleavy, Kevin James
(86) International application number: PCT/JP2011/076584
(87) International publication number: WO 2013/073041

(56) References cited:
- WO-A2-2008/132176
- WO-A2-2009/093213
- US-A1- 2007 134 690
- Agilent Technologies: "Microarray ordering guide", , 26 May 2009 (2009-05-26), pages 1-8, XP002741865, Retrieved from the Internet: URL:https://www.usc.es/export/sites/defaul t/gl/investigacion/riaidt/secuenciacion/ag ilent/descargas/Microarray_Ordering_Guide. pdf [retrieved on 2015-07-07]
- CHRISTOPHER J EDWARDS: "Molecular Profile of Peripheral Blood Mononuclear Cells from Patients with Rheumatoid Arthritis", | M O L M E D, vol. 13, no. 1-2, 1 January 2007 (2007-01-01), pages 40-58, XP055200659, DOI: 10.2119/2006-000056.Edwards
- BATLIWALLA F M ET AL: "PERIPHERAL BLOOD GENE EXPRESSION PROFILING IN RHEUMATOID ARTHRITIS", GENES AND IMMUNITY, NATURE PUBLISHING GROUP, GB, vol. 6, no. 5, 1 August 2005 (2005-08-01), pages 388-397, XP008063573, ISSN: 1466-4879, DOI: 10.1038/SJ.GENE.6364209
- Takeuchi, Tsutomu: "Identification and characterization of pathogenesis related molecules in early rheumatoid arthritis by DNA microarray", , 2014, pages 1-5, XP002741866, Retrieved from the Internet: URL:http://koara.lib.keio.ac.jp/xoonips/mo dules/xoonips/detail.php?koara_id=KAKEN_23 390259seika [retrieved on 2015-07-07]

## Description

### FIELD OF THE INVENTION

The present invention relates to a method for determining a progression of rheumatoid arthritis in a subject or determining a therapeutic effect of a therapeutic drug for rheumatoid arthritis. More specifically, the method measures and normalizes an amount of expression of a biomarker gene in blood collected from a subject and then determines a rheumatoid arthritis activity indicator in the subject on the basis of a gene expression profile prepared from the amount of expression.

### BACKGROUND OF THE INVENTION

Rheumatoid Arthritis (RA), a typical rheumatic disease, is a systemic connective tissue disorder having chronic polyarthritis as the major symptom and is one type of autoimmune diseases. Rheumatoid arthritis frequently occurs among women of 30 to 60 years old, and it is estimated that the number of patients suffering from this disease is about 700,000 in Japan. The condition of rheumatoid arthritis includes angiogenesis in the arthrosynovial membrane, inflammatory cell infiltration, synoviocyte proliferation, and cartilage and bone destruction. In rheumatoid arthritis, arthritis progresses while repeating amelioration and exacerbation and gradually destroys cartilages and bones, thereby inhibiting daily activities of patients with rheumatoid arthritis and lowering their quality of life (QOL).

In the clinical rheumatoid arthritis, the activity of the condition and progression of rheumatoid arthritis in patients has been evaluated by calculating a specific indicator in order to provide the best therapy to those patients. By way of example, in evaluating this indicator, the activity of the condition and progression of rheumatoid arthritis has been scored by combining various evaluation items including the self-evaluation of pain by a patient, the examination and measurement of joints (pain joint count, tender joint count, swollen joint count, etc.) and blood examination (erythrocyte sedimentation rate, rheumatoid factor, CRP, etc.). Based on the scored indicator, the degree of disorder of the patient is evaluated. It is also reported that an urinary interleukin-1 receptor antagonist can be used as an indicator (Japanese Patent No. 3530239).

WO 2009/093213 teaches a method for predicting outcome of concomitant chemo-radiotherapy of a subject suffering from a brain tumor by measuring an expression level of gene clusters associated with tumor resistance to the concomitant chemo-radiotherapy treatment. The gene clusters are selected from the group comprising HOX genes, G1 3 genes, G1 8 genes, G25 genes, G07 genes and G14 genes. WO 2008/132176 teaches a method of predicting the responsiveness of a patient to a TNF blocking drug by assessing an expression level of at least one gene in a synovial sample from the patient and predicting the responsiveness of the patient to the treatment with the TNF blocking agent.

US 2007/0134690 teaches a method of diagnosing systemic onset juvenile idiopathic arthritis (SoJIA) by determining the expression level of a biomarker in blood. US 2007/0134690 mentions that the gene FAM20A is differentially expressed in a patient with SoJIA but does not consider it to be a viable candidate for diagnosing SoJIA. US 2007/0134690 does not list FAM20A as one of the 50 viable candidates and indicates that FAM20A does not meet its criteria for a statistically significant correlation with SoJIA.

Agilent Technologies: microarray ordering guide (26, May, 2009) provides a catalogue of microarray products from Agilent Technologies. Edward et al. ("Molecular profile of peripheral blood mononuclear cells from patients with rheumatoid arthritis," Mol. Med., vol. 13, pp.40-58, 2007) discloses identifying rheumatoid arthritis disease-related genes in peripheral blood by performing a global gene expression analysis using an oligonucleotide array. Batliwalla et al. ("Peripheral blood gene expression profiling in rheumatoid arthritis," Genes Immun., vol. 6, pp.388-397, 2005) discloses a method of gene expression profiling of peripheral blood in rheumatoid arthritis patients using microarrays.

### SUMMARY OF THE INVENTION

In the case of evaluating the activity of the condition and progression of rheumatoid arthritis using the abovementioned indicator, patients' self-evaluation of pains is necessarily involved as described above, and therefore patients' subjective judgment is required. However, since patients' own evaluation of pains and the like fluctuates depending on individuals and observers, it is difficult to make objective evaluation of the activity. Moreover, it is also difficult to comprehensively evaluate the condition and progression of patients using currently-used markers in blood alone. Accordingly, in order to diagnose rheumatoid arthritis disease and estimate a prognosis after treatment, there is a need to find a simple marker for the disease with high reproducibility and develop a method for directly determining the degree of the disease.

The present invention was made under the abovementioned circumstances, and the purpose of the invention is to find a simple biomarker with high reproducibility with regard to a method for evaluating the activity of the condition and progression of rheumatoid arthritis disease and to provide an effective evaluation method therefor.

In order to solve the above mentioned problems, the present inventors paid attention to the amount of expression of the *FAM20A* gene originated from human patients with rheumatoid arthritis disease and thereby found that the amount of expression of the *FAM20A* gene changes in proportion to the level of the activity of the condition of rheumatoid arthritis disease. Accordingly, as a result of intensive studies on the amount of expression of the *FAM20A* gene, they found that *FAM20A* in blood collected from a subject could be a diagnostic marker for rheumatoid arthritis disease as well as a marker for the effect of administering therapeutic drugs and that the activity of the condition and progression of rheumatoid arthritis disease could be evaluated by determining the amount of expression of the *FAM20A* gene.

More specifically, according to the present invention, provided is a method for determining a progression of rheumatoid arthritis using a rheumatoid arthritis linked indicator in a subject or determining a therapeutic effect of a therapeutic drug for rheumatoid arthritis in a subject, comprising measuring an amount of expression of the *FAM20A* gene in blood derived from the subject, a) comparing the measured amount of expression of the FAM20A gene in the peripheral blood derived from the subject with a gene expression profile that is prepared based on an amount of expression of the FAM20A gene in peripheral blood collected from a patient with rheumatoid arthritis, when a progression of rheumatoid arthritis in a subject is determined ; or b) comparing the measured amount of expression of the FAM20A gene in the peripheral blood derived from the subject after administration of a therapeutic drug with a gene expression profile that is prepared based on an amount of expression of the FAM20A gene in peripheral blood collected from the subject prior to administration of the therapeutic drug , when a therapeutic effect of a therapeutic drug for rheumatoid arthritis is determined, and wherein said gene expression profile shows a positive correlation between an amount of expression of the FAM20A gene and an indicator of a progression of rheumatoid arthritis in a subject, estimating the value of the indicator in the subject on the basis of the comparing step, and determining a progression of rheumatoid arthritis in a subject or determining a therapeutic effect of a therapeutic drug for rheumatoid arthritis from the estimated value of the indicator.

In this constitution, the value of a rheumatoid arthritis-linked indicator can be determined in a subject simply by taking a blood sample without placing a special burden on the subject. Moreover, in this constitution, the value of a rheumatoid arthritis-linked indicator can be determined in a low invasive and simple manner.

Since the value of a rheumatoid arthritis-linked indicator can be determined by measuring the amount of expression of the *FAM20A* gene in blood, reproducibility is high, reliability is also high as compared with the conventional method including patients' self-evaluation, and examination can be made with ease. Moreover, the use of the method according to the present invention enables to determine the therapeutic effect of a therapeutic drug for rheumatoid arthritis. This method is useful because subjective judgment of pains and the like by patients is not required such that the effect can be determined objectively.

Furthermore, according to one embodiment of the present invention, in the abovementioned method, the indicator is preferably selected from the group consisting of DAS28-CRP, DAS28-ESR, DAS44, EULAR improvement criteria, CDAI, SDAI, ACR and serum CRP concentration. In this case, the indicator shows the progression of rheumatoid arthritis or enables to determine a therapeutic effect of therapeutic drugs for rheumatoid arthritis.

Furthermore, according to another embodiment of the present invention, in the abovementioned method, the indicator shows high activity of progression when the amount of expression of the *FAM20A* gene is large.

Furthermore, according to another embodiment of the present invention, in the abovementioned method, the *FAM20A* gene preferably consists of (i) a base sequence according to SEQ ID NO: 1 or (ii) a base sequence that hybridizes with the base sequence according to SEQ ID NO: 1 under stringent conditions, the base sequence being a base sequence encoding a protein having a function equivalent to that of a protein consisting of an amino acid sequence according to SEQ ID NO: 2.

Furthermore, according to another embodiment of the present invention, in the abovementioned method, the subject is preferably suffering from rheumatoid arthritis. In this case, the measuring measures amounts of expression of the *FAM20A* gene derived from blood before and after administering a therapeutic agent for rheumatoid arthritis to the subject, wherein a therapeutic effect of the therapeutic agent for rheumatoid arthritis is determined by analyzing the amounts of expression of the *FAM20A* gene in blood before and after administration using the gene expression profile.

The features and marked operation and effects of the present invention other than those described above will be obvious to those skilled in the art by referring to detailed description of the invention and drawings as shown below.

### BRIEF DESCRIPTION OF THE DRAWINGS

Fig. 1 is a graph showing the analysis of cells expressing the *FAM20A* gene according to the present invention.
Fig. 2 is a scatter plot graph showing the analysis results of the correlation between the amount of expression of the *FAM20A* gene according to the present invention and DAS28-CRP as well as DAS28-ESR.
Fig. 3A shows changes in the amount of expression of the *FAM20A* gene according to the present invention before and after administering methotrexate.
Fig. 3B shows changes in the amount of expression of the *FAM20A* gene according to the present invention before and after administering infliximab.
Fig. 4 is a graph showing the analysis of expression of the *FAM20A* gene according to the present invention in normal individuals and patients with inflammatory diseases.
Fig. 5A is a graph showing the correlation between microarrays of the *FAM20A* gene according to the present invention and PCR as a result of analyzing expression.
Fig. 5B is a graph showing the significant difference between a normal individual and a patient with rheumatoid arthritis with regard to the amount of expression of the *FAM20A* gene according to the present invention.
Fig. 5C is a graph showing the correlation between DAS28-CRP, which is an indicator for the activity of disease, and CRP, which is clinical information, with regard to the amount of expression of the *FAM20A* gene according to the present invention.

### DETAILED DESCRIPTION OF THE INVENTION

In the following, one embodiment of the present invention and examples are given with reference to drawings.

As described above, the method according to the present embodiment for determining the progression of rheumatoid arthritis in a subject or determining a therapeutic effect of a therapeutic drug for rheumatoid arthritis in a subject is characterized by comprising a step for measuring the amount of expression of the *FAM20A* gene in blood originated from the abovementioned subject, a step a) comparing the measured amount of expression of the FAM20A gene in the peripheral blood derived from the subject with a gene expression profile that is prepared based on an amount of expression of the FAM20A gene in peripheral blood collected from a patient with rheumatoid arthritis, when a progression of rheumatoid arthritis in a subject is determined ; or b) comparing the measured amount of expression of the FAM20A gene in the peripheral blood derived from the subject after administration of a therapeutic drug with a gene expression profile that is prepared based on an amount of expression of the FAM20A gene in peripheral blood collected from the subject prior to administration of the therapeutic drug, when a therapeutic effect of a therapeutic drug for rheumatoid arthritis is determined, and wherein said gene expression profile shows a positive correlation between an amount of expression of the FAM20A gene and an indicator, a step for estimating the value of the abovementioned indicator in the abovementioned subject on the basis of the comparing step, and a step of determining a progression of rheumatoid arthritis in a subject or determining a therapeutic effect of a therapeutic drug for rheumatoid arthritis from the estimated value of the indicator.

In one embodiment of the present invention, the amount of expression of the *FAM20A* gene changes in direct proportion to an indicator of the activity of rheumatoid arthritis and particularly reflects variations in the indicator of activity as described in examples below. As used herein, the term "gene marker" is used substantially as the same meaning as "gene," is an indicator for evaluating the condition or operation of a certain object and refers to a gene-linked substance that correlates with the amount of expression of a certain gene. By way of example, included are a gene itself, mRNA, which is a transcript, a peptide, which is a translated substance, and a protein, which is the final product of expression of a gene. The term "relative expression frequency" of a gene refers to the normalized amount of expression of the gene and includes the amount of expression on the transcription level of the gene (in the case of a transcript) or the amount of expression on the translation level (in the case of a polypeptide, a protein or the like).

In the present embodiment, the term "rheumatoid arthritis-linked indicator" includes DAS28-CRP, DAS28-ESR, DAS44, EULAR improvement criteria, CDAI, SDAI, ACR and serum CRP concentration, yet the indicator is not particularly limited to those as far as it can be expressed by digitizing and scoring the degree of the condition and progression of rheumatoid arthritis as well as the activity thereof.

DAS (Disease Activity Score) is an evaluation method recommended by DAS (Disease Activity Score), which is an evaluation method recommended by EULAR (European League Against Rheumatism). Moreover, DAS28 is a DAS in which the number of joints to be evaluated has been narrowed down to 28 in order to facilitate its use in daily medical examination and treatment. In DAS28, those 28 joints are measured for four items, i.e., (1) tender joint count, (2) swollen joint count, (3) hourly value of erythrocyte sedimentation rate or CRP (mg/dl) and (4) overall evaluation of condition and then digitized based on a prescribed formula to calculate the absolute value of the activity of disease. When the erythrocyte sedimentation rate is used, it is referred to as DAS28-ESR, and when the CRP value is used, it is referred to as DAS28-CRP. In the present examples, both DAS28-ESR and DAS28-CRP can be used as DAS28. Moreover, DAS44 is used for measuring 44 joints. In the present embodiment, while DAS28-ESR and DAS28-CRP change in proportion to variations in the amounts of expression of the *FAM20A* gene, the standard values of the amounts of expression (standard amounts of expression) of the *FAM20A* gene corresponding to specific DAS28-CRP, DAS28-ESR and the like can be altered appropriately in accordance with experimental conditions and, therefore, are not particularly limited to any specific numerical values. In DAS28-ESR, the activity of disease is evaluated high when the value is 5.1 (4.1) or higher, moderate when 3.2 (2.7) < DAS28-ESR =< 5.1 (4.1), low when 2.6 (2.3) < DAS28-ESR =< 3.2 (2.7) and as remission when it is 2.6 (2.3) or below (the descriptions in the parentheses correspond to DAS28-CRP). As used herein, the term "remission" refers to the state in which the condition of disease has been alleviated temporarily or continuously or has disappeared apparently.

Furthermore, in one embodiment of the present disclosure, the EULAR improvement criteria can be used when the value of a rheumatoid arthritis-linked indicator is determined in a subject, wherein the basis of the EULAR improvement criteria is DAS28. The is evaluated at three stages, i.e., good, moderate and no response in combination of two DAS28 values, i.e., a value after therapy as compared with a value before therapy (i.e., a value after administering a drug as compared with a value before administering the drug). In the present embodiment, the therapeutic effect can be evaluated by any of the abovementioned three stages for the subject in proportion to variations in the amount of expression of the *FAM20A* gene. In the present embodiment, while the EULAR improvement criteria change in proportion to variations in the amount of expression of the *FAM20A* gene, the standard value of the amount of expression (standard amount of expression) of the *FAM20A* gene corresponding to specific EULAR improvement criteria can be altered appropriately in accordance with experimental conditions and, therefore, is not particularly limited to any specific numerical value. By way of example, once the amount of change in the expression of the *FAM20A* gene corresponding to "moderate" is set, the amounts of change in the expression of the *FAM20A* gene corresponding to "good" and "no response" can also be set appropriately on the basis of the analysis of a gene expression profile.

In one embodiment of the present disclosure, when the value of a rheumatoid arthritis-linked indicator is determined in a subject, SDAI (Simple Disease Activity Index) can also be used as the indicator. SDAI was developed as an evaluation method for facilitating calculation at a clinical site because the abovementioned calculation formula for DAS was so complicated that it could not easily be calculated at a clinical site. This SDAI is calculated on the basis of tender joint count, swollen joint count, patients' general evaluation and doctors' general evaluation, wherein joints to be evaluated are the same as those in DAS28. In SDAI, the activity of disease is classified and evaluated high when the value is 26 or higher, moderate when 11 < SDAI =< 26, low when 3.3 < SDAI =< 11 and as remission when it is 3.3 or below. In the present embodiment, while the value of SDAI changes in proportion to variations in the amount of expression of the *FAM20A* gene, the standard value of the amount of expression (standard amount of expression) of the *FAM20A* gene corresponding to a specific value of SDAI can be altered appropriately in accordance with experimental conditions and, therefore, is not particularly limited to any specific numerical value. By way of example, once the amount of expression of the *FAM20A* gene corresponding to a specific value of SDAI showing the moderate activity of disease is set, the amounts of expression of the *FAM20A* gene corresponding to specific values showing the other activities such as high activity of disease and low activity of disease can also be set appropriately on the basis of the analysis of a gene expression profile.

Furthermore, in one embodiment of the present invention, when the value of a rheumatoid arthritis-linked indicator is determined in a subject, CDAI (Clinical Disease Activity Index) can also be used as the indicator. CDAI is found by removing CRP from SDAI. In CDAI, the activity of disease is classified and evaluated high when the value is 22 or higher, moderate when 10 < CDAI =< 22, low when 2.8 < CDAI =< 10 and as remission when it is 2.8 or below. In the present embodiment, while the value of CDAI changes in proportion to variations in the amount of expression of the *FAM20A* gene, the standard value of the amount of expression (standard amount of expression) of the *FAM20A* gene corresponding to a specific value of CDAI can be altered appropriately in accordance with experimental conditions and, therefore, is not particularly limited to any specific numerical value. By way of example, once the amount of expression of the *FAM20A* gene corresponding to a specific value of CDAI showing the moderate activity of disease is set, the amounts of expression of the *FAM20A* gene corresponding to specific values showing the other activities such as high activity of disease and low activity of disease can also be set appropriately on the basis of the analysis of a gene expression profile.

In one embodiment of the present invention, when the value of a rheumatoid arthritis-linked indicator is determined in a subject, ACR can also be used as the indicator. ACR refers to ACR improvement criteria, which are classification criteria set by the American College of Rheumatology to show the pathological level of rheumatoid arthritis. The ACR improvement criteria are defined by seven items of an ACR core set. The ACR core set consists of the seven items, i.e., (1) tender joint count, (2) swollen joint count, (3) evaluation of disease by patients, (4) general evaluation of the activity of disease by patients, (5) general evaluation of the activity of disease by doctors, (6) evaluation of physical function by patients, and (7) acute phase reactants. The ACR improvement criteria is, for example, determined as "ACR criteria improved by 20% (ACR20)" in the case that both of the abovementioned (1) and (2) are improved by 20% or more and three out of the remaining five items, i.e., the abovementioned (3) through (7) are improved by 20% or more after treatment as compared with pre-treatment (i.e., after administering a drug as compared with before administering the drug). Similarly, in the case that ACR is improved by 50% and 70%, ACR50 and ACR70 are used as criteria, respectively. As used herein, the simple description of "ACR" indicates the "ACR improvement criteria" unless otherwise specified. In the present embodiment, while the ACR criteria change in proportion to variations in the amount of expression of the *FAM20A* gene, the standard value of the amount of expression (standard amount of expression) of the *FAM20A* gene corresponding to specific ACR criteria can be altered appropriately in accordance with experimental conditions and, therefore, is not particularly limited to any specific numerical value. By way of example, once the amount of change in the expression of the *FAM20A* gene corresponding to ACR50 is set, the amounts in the expression of the *FAM20A* gene corresponding to ACR20 and ACR70 can also be set appropriately on the basis of the analysis of a gene expression profile.

In one embodiment of the present disclosure, when the value of a rheumatoid arthritis-linked indicator is determined in a subject, serum CRP concentrations can also be used as the indicator. The normal serum CRP value is 0.3mg/dl. In the present embodiment, while the serum CRP value changes in proportion to variations in the amount of expression of the *FAM20A* gene, the standard value of the amount of expression (standard amount of expression) of the *FAM20A* gene corresponding to a normal serum CRP value can be altered appropriately in accordance with experimental conditions and, therefore, is not particularly limited to any specific numerical value.

Furthermore, in one embodiment of the present invention, the *"FAM20A* gene" consists of (i) a base sequence according to SEQ ID NO: 1 or (ii) a base sequence that hybridizes with the base sequence according to SEQ ID NO: 1 under stringent conditions, the base sequence being a base sequence encoding a protein having a function equivalent to that of a protein consisting of an amino acid sequence according to SEQ ID NO: 2. It has been known that *FAM20A* is a secreted protein.

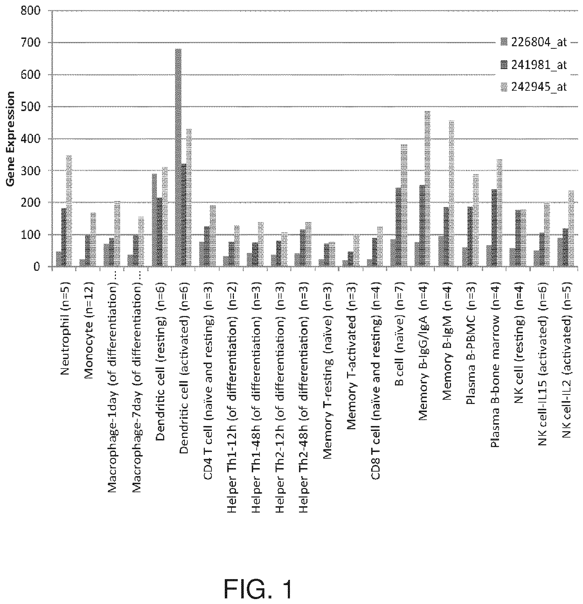

For the purpose of elucidating the biological significance of *FAM20A,* the present inventors analyzed immune cells contained in blood using a microarray data set by immune cells obtained from NCBI GEO (Gene Expression Omnibus), a publicly available microarray database, in order to find which cell is the source of expression of *FAM20A.* Fig. 1 shows the result. It was demonstrated that the *FAM20A* gene was highly expressed in dendritic cells and B cells. It has been known that both dendritic cells, which are antigen-presenting cells, and B cells, which produce antibodies, contribute significantly to the condition of rheumatism. Accordingly, it was biologically assumed that the expression of *FAM20A* in peripheral blood was highly likely involved in the condition of rheumatoid arthritis.

In one embodiment of the present disclosure, a method for determining the progression of rheumatoid arthritis in a subject or determining a therapeutic effect of a therapeutic drug for rheumatoid arthritis in a subject comprises measuring the amount of expression of the *FAM20A* gene contained in blood collected from the subject. In the determination method according to the present embodiment, the test specimen includes blood collected from a subject, and whole blood, peripheral blood or serum isolated from the blood may also be used as a test specimen. In this case, the method for obtaining serum is not particularly limited, and a wide variety of conventionally known methods may be used (e.g., a method for isolating serum obtained from blood as a specimen for clinical examination). For example, serum may be produced from supernatant obtained by allowing a blood specimen to stand or supernatant obtained by means of centrifugation. Alternatively, a test specimen may be serum in supernatant obtained by placing collected blood in a test tube or the like and then centrifuging it at room temperature under prescribed conditions.

In one embodiment of the present invention, the method for determining the progression of rheumatoid arthritis in a subject or determining a therapeutic effect of a therapeutic drug for rheumatoid arthritis in a subject facilitates the acquisition of a specimen in that blood originated from a subject can easily be examined as a specimen as compared with a method for collecting and examining synovial fluid and is also advantageous in that a test specimen used in an ordinary clinical examination can be used. Since synovial fluid is collected by an invasive method, patients might be significantly burdened with pain or the like, and there is also some possibility that complications might occur including bleeding and infectious diseases; therefore the examination of synovial fluid is not very recommendable as a routine test method. On the other hand, the abovementioned problems can be solved if the amount of expression of the *FAM20A* gene is measured using blood as a specimen as shown in the present invention, and the progression of rheumatoid arthritis in a subject or determining a therapeutic effect of a therapeutic drug for rheumatoid arthritis can easily be determined in a subject. As used herein, the amount of *FAM20A* refers to the amount of expression of the *FAM20A* gene unless otherwise specified.

In one embodiment of the present invention, the method for determining the progression of rheumatoid arthritis in a subject or determining a therapeutic effect of a therapeutic drug for rheumatoid arthritis in a subject uses the *FAM20A* gene in collected blood as a marker of an indicator for the activity of rheumatoid arthritis. The result of measuring the amount of expression of the *FAM20A* gene obtained by the method according to the present invention enables to determine progression of rheumatoid arthritis in a subject or determining a therapeutic effect of a therapeutic drug for rheumatoid arthritis and furthermore enables to diagnose rheumatoid arthritis and determine the therapeutic effect of therapeutic drugs for rheumatoid arthritis.

In one embodiment of the present invention, when the therapeutic effect of a therapeutic drug for rheumatoid arthritis is determined using the method according to the present invention, blood is collected from a subject with rheumatoid arthritis before and after administering the therapeutic drug for rheumatoid arthritis, and then the amount of expression of the *FAM20A* gene in each of the collected blood is measured before and after administration. Subsequently, the amount of expression of the *FAM20A* gene in each of the blood is compared with a gene expression profile that is prepared in advance and correlates with a rheumatoid arthritis-linked indicator.

In the present embodiment, as a result of determining the therapeutic effect of a rheumatoid arthritis by the abovementioned method, it has been demonstrated that variations in the expression of the *FAM20A* gene before and after administering the therapeutic drug is closely related to the evaluation of the actual therapeutic effect. For example, the relationship between the result of measuring the expression of the *FAM20A* gene and the evaluation by the EULAR improvement criteria can clearly reflects the level of the therapeutic effect (See examples below).

In the present invention, "therapeutic drugs for rheumatoid arthritis" or "drugs for treating rheumatoid arthritis" are not particularly limited and include conventionally well-known therapeutic drugs and all kinds of therapeutic drugs that will be developed in the future. For example, the conventionally well-known therapeutic drugs include biological preparations, non-steroidal anti-inflammatory drugs (anti-inflammatory analgesics), steroidal drugs and immune-suppressants. The biological preparations include chimeric anti-TNF-alpha antibody preparations, soluble TNF receptors, fully human anti-TNF-alpha antibody preparations and anti-IL-6 receptor antibody preparations. The non-steroidal anti-inflammatory drugs include prostaglandin synthesis inhibitors. More specifically, the therapeutic drugs for rheumatoid arthritis according to the present invention include, but are not limited to, methotrexate (MTX), infliximab (IFX), etanercept (ETN), tocilizumab (TCZ), adalimumab (ADA) and abatacept (ABT).

In one embodiment of the present invention, the progression of rheumatoid arthritis in a subject or determining a therapeutic effect of a therapeutic drug for rheumatoid arthritis can be determined by comparatively analyzing the amount of expression of the *FAM20A* gene in a subject and a gene expression profile that is prepared in advance from the amount of expression of the *FAM20A* gene in a sample group of various diseases and health conditions including patients with rheumatoid arthritis, normal individuals and administration or no administration of various therapeutic drugs for rheumatoid arthritis. Alternatively, the value of a rheumatoid arthritis-linked indicator may be determined by comparatively analyzing a gene expression profile of a subject (including normal individuals and patients with rheumatoid arthritis) before administering a therapeutic drug for rheumatoid arthritis and a gene expression profile of the same subject after administering the therapeutic drug for rheumatoid arthritis. In this case, the abovementioned sample group of various diseases and health conditions may be sampled by means of classification on the basis of various elements such as gender, age, type of therapeutic drugs and time after administering a therapeutic drug. As to the gene expression profile that is prepared in advance, the number of accumulated samples is preferably as large as possible in that the accuracy of the determination method according to the present invention increases. Moreover, in the present disclosure, the constitution may be such that data on such accumulated samples and the gene expression profile that is prepared in advance can be stored in an optional database. In other words, the present disclosure can provide a database for storing such data and an analyzer for reading and executing such data and programs needed for comparative analysis. Such an analyzer can determine easily and at all times the value of a rheumatoid arthritis-linked indicator in a subject simply by retrieving accumulated data from the database and comparing it with measured data of the subject.

In the present embodiment, the value of a rheumatoid arthritis-linked indicator in a subject can be determined and analyzed by comparing the measured amount of expression of the *FAM20A* gene with a gene expression profile prepared on the basis of the amount of expression of the *FAM20A* in various diseases and health conditions, and as to a period after administering a therapeutic drug, any optional period can be set that is clinically significant in evaluating the condition of rheumatoid arthritis disease.

Furthermore, in the present embodiment, it is preferred that the abovementioned gene expression profile show the positive correlation between the amount of expression of the *FAM20A* gene and the value of a specific rheumatoid arthritis-linked indicator. Accordingly, by way of example, if the amount of expression of the *FAM20A* gene in blood originated from a subject is higher than the standard amount of expression, the indicator shows the activity of progression higher than the standard value.

In one aspect of the present disclosure, as a method for preparing and generating a gene expression profile and analyzing data, any optional analysis means used in the field of microbiology or bioinformatics can be used including cluster analysis and various types of algorisms.

### (Experiment method)

The following describes an experiment method and substances used for demonstrating the effect of a gene marker according to one embodiment of the present invention as well as the definition thereof. Although the following experiment method is used in the present embodiment, the similar result can be achieved even when an experiment method other than the method shown below is used.

### (Quantification of gene)

Furthermore, in the present embodiment, when the gene marker is a gene or a transcript of a gene (mRNA), the amount of expression of the gene can be measured (quantified) by measuring the amount of mRNA by means of various types of microbiological methods including DNA chips, microarray techniques, real-time PCR, Northern blotting techniques, dot blotting techniques and quantitative RT-PCR (quantitative Reverse Transcription-Polymerase Chain Reaction).

The primer used in the quantitative RT-PCR method is not particularly limited as far as it can specifically detect a gene or a gene marker and is preferably an oligonucleotide having 12 to 26 bases. Its base sequence is determined on the basis of sequence information about each human gene. A primer having the determined sequence can be produced using a DNA synthesizer, for example.

On the other hand, when the gene marker is a translated substance (polypeptide) or final product (protein) of a gene, the amount of expression of the gene marker can be measured by means of Western blotting techniques, ELISA techniques, etc. using a polyclonal antibody, a monoclonal antibody and the like specific to the gene marker, yet the method includes various other techniques such as RIA (Radioimmunoassay) and EIA (Enzyme Immunoassay).

The "gene" refers to genetic information represented by base sequences of RNA, DNA, etc. Also included are ortholog genes preserved among species such as human, mice and rats. The gene may be not only one that encodes a protein but one that functions as RNA, DNA or the like as well. The gene generally encodes a protein based on its base sequence but may also be one that encodes a protein whose biological function is equivalent to that of the abovementioned protein (e.g., homologues (homologues, splicing variants), mutants, derivatives). For example, also included is a gene encoding a protein that is slightly different in terms of base sequence from a protein represented by a base sequence according to genetic information, wherein its base sequence hybridizes with a sequence complimentary to the base sequence according to genetic information.

"RNA" includes not only single-stranded RNA but single-stranded RNA having a sequence complimentary thereto and double-stranded RNA constituted therefrom as well. Also included are totalRNA, mRNA and rRNA.

"DNA chips" and "DNA arrays" have a structure in which probe DNA is arranged on a substrate and are used for measuring the expression of multiple genes by means of hybridization. They include not only those used for optically measuring the amount of expression but those that outputs the amount of expression electrically as well. As "DNA chips," GeneChip® manufactured by Affymetrix, Inc. may be used, for example. As "DNA arrays," CodeLink Expression Bioarray® manufactured by Amersham Biosciences Corp. may be used. DNA arrays include DNA macroarrays as well as DNA microarrays.

The "amount of expression" includes not only values obtained by directly measuring the amount of expression of a gene but values obtained by conversion by means of prescribed calculation, statistical techniques, etc. as well. Moreover, the "amount of gene expression," "expression signals," "gene expression signals," "values of expression signals," "values of gene expression signals," "gene expression data, "expression data," and the like are interchangeably used to show values that reflect the expression of individual genes.

The "gene expression" refers to the mode of expression of a gene in the living body expressed by the amount of expression of the gene and includes both the case in which it is expressed by the amount of expression of one gene and the case in which it is expressed by a plurality of genes. Additionally, the term "expression" is also used interchangeably to show the mode of expression of a gene in the living body.

### Examples

In the following, the present invention will be described in more detail with reference to examples.

### (Method for searching for and selecting maker gene)

The following describes a method for searching for and selecting a marker gene according to the present disclosure.

The present inventors searched for a marker gene using peripheral blood samples of 212 people (402 specimens in total) from patients with rheumatoid arthritis who visited the Department of Rheumatology/Clinical Immunology, Saitama Medical Center, Saitama Medical University, received a written explanation about participation in studies and gave consent. The breakdown of those samples is shown below.

**Table 1 Breakdown of samples**

| Classification | Number of samples |
|---|---|
| MTX0w | 29 |
| MTX4w | 23 |
| IFX0w | 103 |
| IFX2w | 91 |
| IFX22w | 54 |
| IFX54w | 20 |
| ETN0w | 52 |
| ETN2w | 20 |
| TCZ0w | 10 |
| Total | 402 |

RNA was extracted from blood of the test subjects using the PAXgene Blood RNA System (manufactured by QIAGEN, Inc.). We measured the yield of the extracted RNA using NanoDrop1000 (manufactured by Thermo Scientific, Inc.) and made sure that there was no decomposition by Bioanalyzer 2100 (Agilent Technologies, Inc.). Next, we amplified cRNA from 250ng of RNA by in vitro transcription reaction using Low RNA Input Linear Amp Kit PLUS, One-Color manufactured by Agilent Technologies, Inc. and at the same time fluorescently labeled it (Cy3 labeling). Subsequently, we hybridized the fluorescently labeled cRNA with Whole Human Genome Microarray 4 x 44k manufactured by Agilent Technologies, Inc. at 65°C for 17 hours. After washing using Gene Expression Wash Buffer manufactured by Agilent Technologies, Inc., we read fluorescent images by Agilent Scanner (manufactured by Agilent Technologies, Inc.) and then digitized the signal intensity of each spot on the fluorescent images using Feature Extraction, image digitization software manufactured by Agilent Technologies, Inc.

We normalized the digitized data under 75 percentile shift normalization using GeneSpring GX11, microarray analysis software manufactured by Agilent Technologies, Inc. We then calculated a Pearson's correlation coefficient and a p value in the test of no correlation between Log 2 (normalized value) of each probe and DAS28-CRP and DAS28-ESR of a subject to extract a gene that correlates with the activity of disease.

We averaged p values calculated for each probe in the test of no correlation with DAS28-CRP and DAS28-ESR and sorted the data by the average values in ascending order to give a probe list of top 30. Table 2 shows the results.

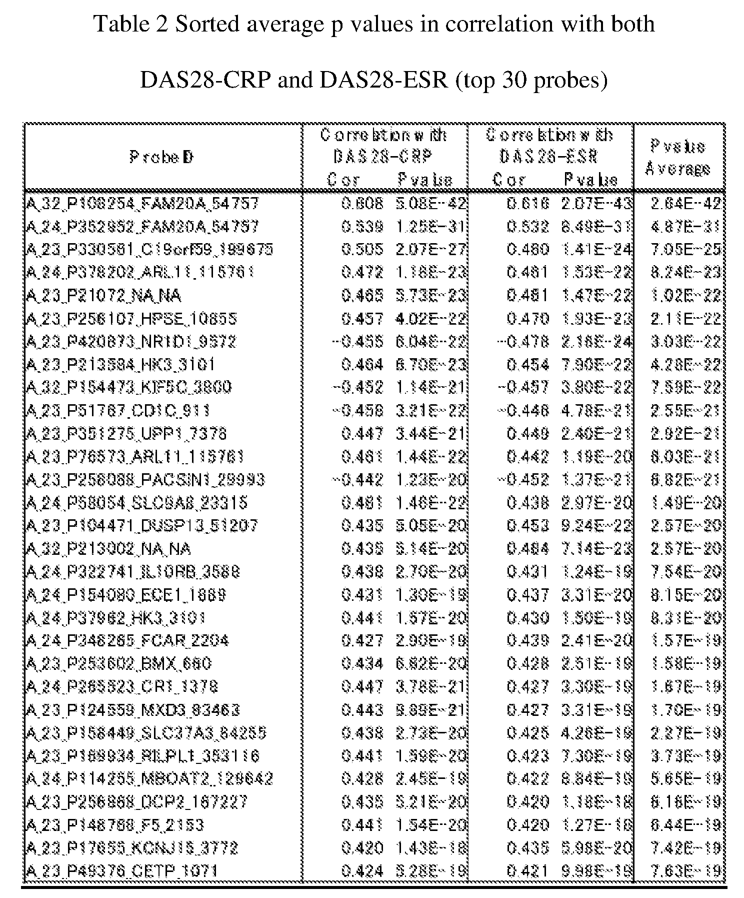

Two probes on the top were probes for detecting transcription products of *FAM20A* (Family with sequence similarity 20, member A). In the gene, expression accelerates in peripheral blood as the activity of rheumatoid arthritis disease increases.

### (Analysis of FAM20A gene)

In the following, the result of analyzing the *FAM20A* gene according to the present invention will be described.

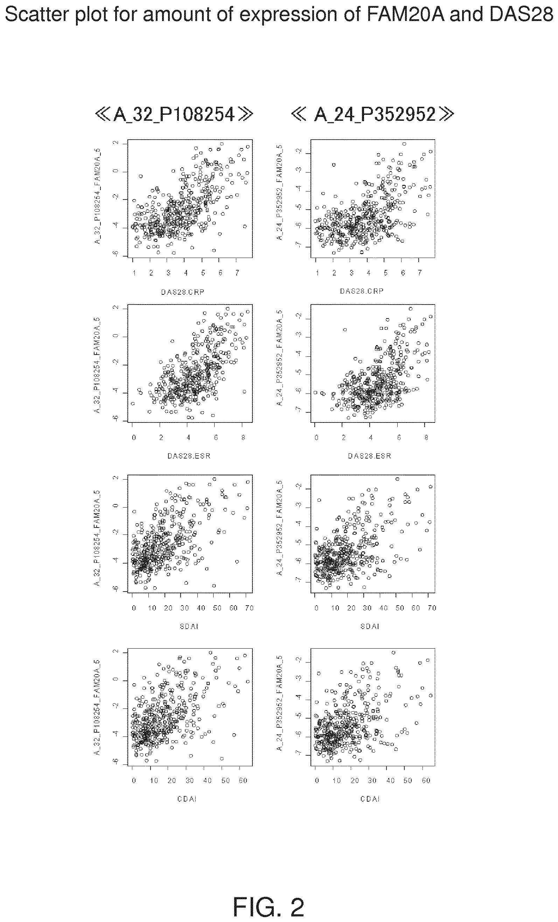

Fig. 2 shows scatter plots between *FAM20A* Log 2 (normalized values) and DAS28-CRP, DAS28-ESR, SDAI and CDAI. Additionally, Table 3 shows the analysis results of the correlation between *FAM20A* and clinical examination items other than DAS28.

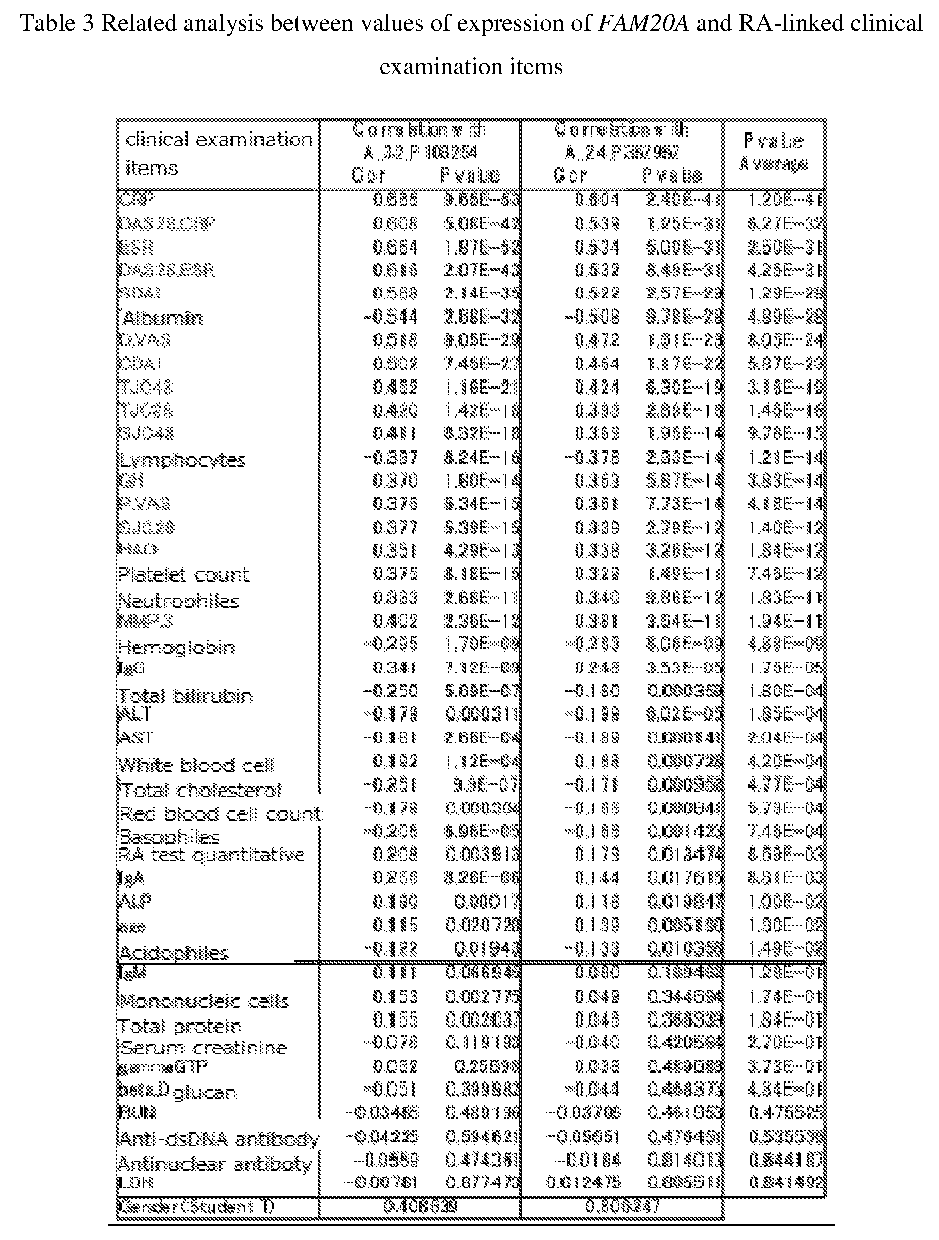

This result shows that there is a significant positive correlation between FAM20A Log 2 (normalized values) and DAS components such as CRP, ESR, TJC, SJC and GH, HAQ (Health Assessment Questionnaire), which is an indicator for evaluating vital functions, and MMP-3, which is believed to increase in rheumatic blood. Accordingly, we believe that by measuring the amount of expression of *FAM20A* in peripheral blood, we can find the activity of rheumatoid arthritis objectively and comprehensively.

### (Changes in expression of FAM20A gene by administering drugs)

We examined how the expression of *FAM20A* changes in the same patient with rheumatoid arthritis before and after drug treatment.

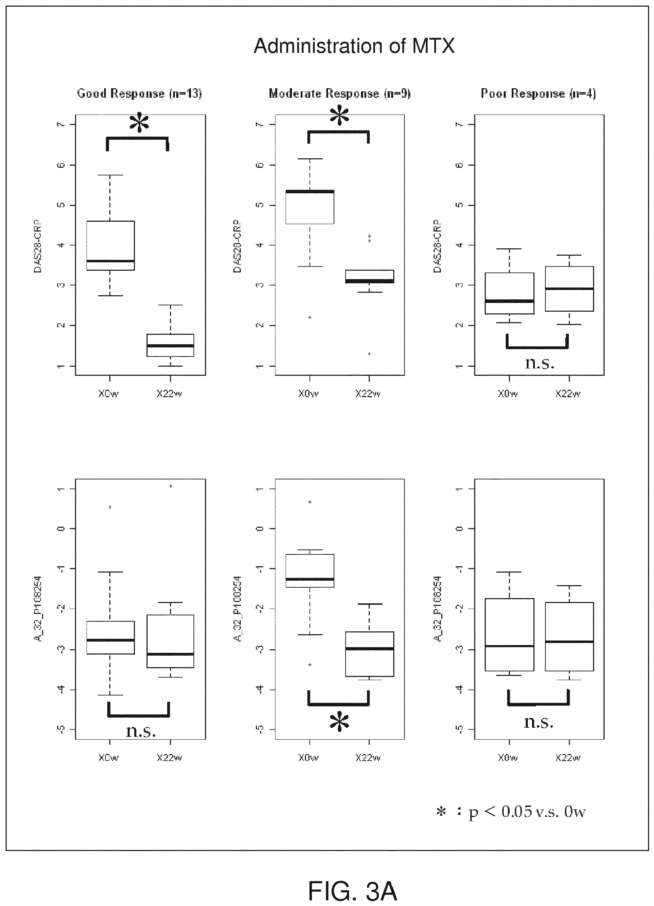

MTX was administered to 26 cases for 22w, and then the result was classified into three groups (good, moderate and poor) on the basis of EULAR improvement criteria, wherein Fig 3A shows changes in the expression *of FAM20A* in each group before and after the administration of MTX. The expression *of FAM20A* declined significantly in 22w as compared with 0w in the moderate response group according to EULAR improvement criteria and also showed the tendency to decline in 22w in the good response group. On the other hand, there was hardly any observable change in the poor response group.

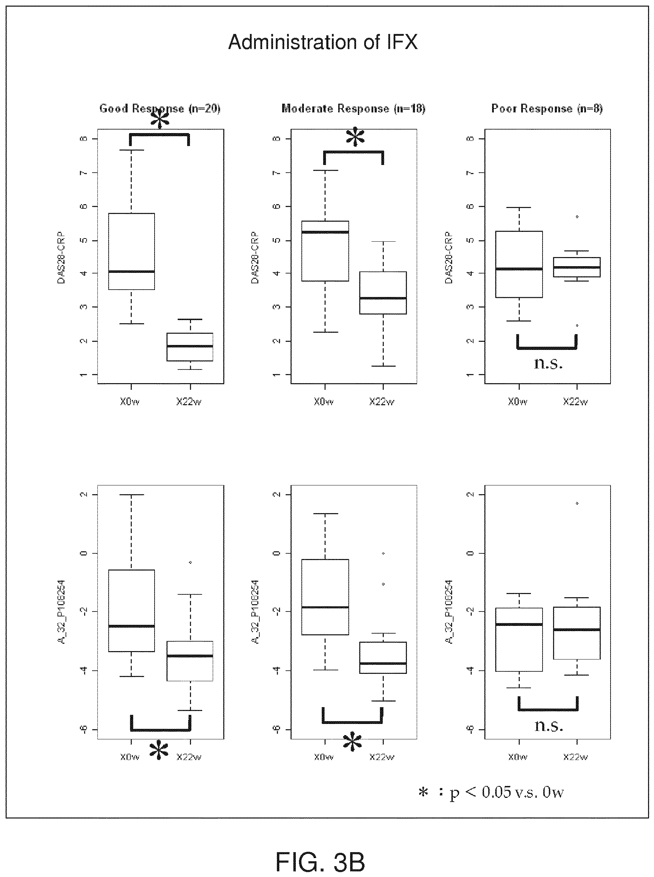

Fig. 3B shows the result after analyzing 46 cases administered with IFX in a manner similar to that described above. The tendency similar to that in the case in which MTX had been administered was observed such that the expression of *FAM20A* declined significantly in 22w in the good and moderate response groups while there was hardly any change in the poor response group. The abovementioned results suggest that the expression of *FAM20A* in peripheral blood can be a marker that reflects the effect of a drug for rheumatoid arthritis.

### (Comparison with normal individuals and other diseases)

We compared the amount of expression *of FAM20A* among patients with rheumatoid arthritis, normal individuals and patients with hepatitis to examine whether or not there was some possibility that *FAM20A* could be a marker for diagnosing rheumatoid arthritis. Samples obtained from patients with hepatitis were used as a control for inflammatory diseases.

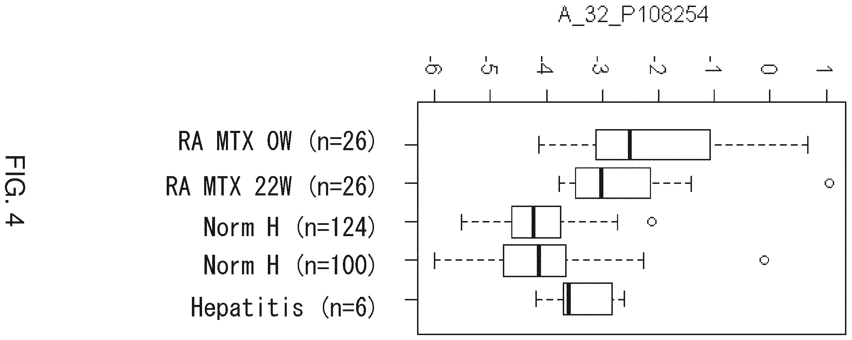

As samples from patients with rheumatoid arthritis, we analyzed MTX 0w and 22w samples in 26 cases administered with MTX as shown in Fig. 3A. As samples from normal individuals, we analyzed peripheral blood samples from 124 people who visited Hishokai Hokkoku Clinic, an incorporated medical institution (Kanazawa City, Ishikawa Prefecture), for the purpose of medical examination as healthy individuals, received a written explanation about participation in studies on the expression of genes in peripheral blood and gave consent (Norm H) as well as peripheral blood samples from 100 people who visited the Public Central Hospital of Matto Ishikawa (Hakusan City, Ishikawa Prefecture), for the purpose of medical examination as healthy individuals, received a written explanation about participation in studies on the expression of genes in peripheral blood and gave consent (Norm M). Additionally, as control samples for inflammatory diseases, we analyzed peripheral blood samples in 6 patients with hepatitis who visited the Department of Gastroenterology, Kanazawa University Hospital, received a written explanation about participation in studies on the expression of genes in peripheral blood and gave consent (Hepatitis). Fig. 4 shows a distribution of *FAM20A* Log 2 (normalized values) in each sample group.

As a result of the student T test, we found that there was a significant difference (p < 0.05) between RA MTX 0w and Norm H, Norm M and Hepatitis as well as between RT MTX 22w and Norm H/Norm M.

### (Analysis by PCR)

With regard to the "correlation between the expression of *FAM20A* in peripheral blood and the activity of rheumatoid arthritis in patients with rheumatoid arthritis" and the "difference in the expression of *FAM20A* in peripheral blood between normal individuals and patients with rheumatoid arthritis," we validated the results by real-time PCR.

From the abovementioned analysis samples, we picked up 12 cases of normal individuals and 15 cases of patients with rheumatism and performed real-time PCR reaction using a Power SYBR Green RNA-to-CT 1-Step Kit manufactured by Applied Biosystems, Inc. We used 20ng of RNA in the experiment. As an internal standard gene for correction, we used GAPDH (Glyceraldehyde 3-Phosphate Dehydrogenase). Primers used for detecting *FAM20A* in the experiment were as follows:
Left: AGCCACATCGCTCAGACAC (SEQ ID NO: 5); and
Right: GCCCAATACGACCAAATCC (SEQ ID NO: 6).

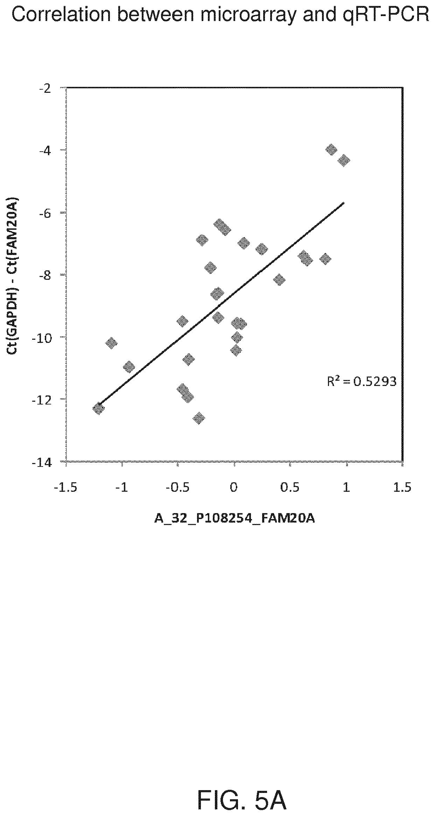

Figs. 5A-C shows the results.

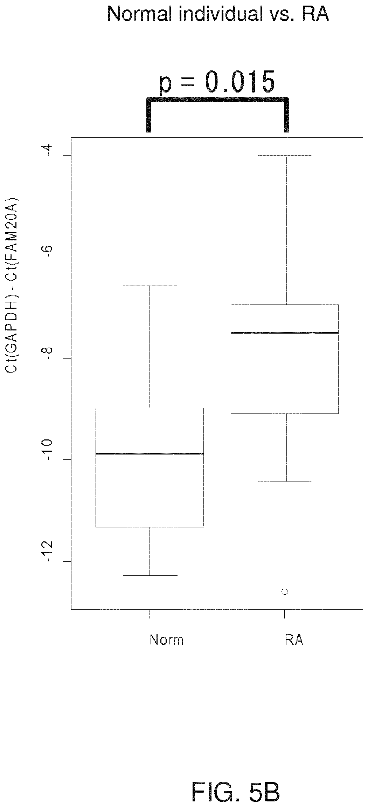

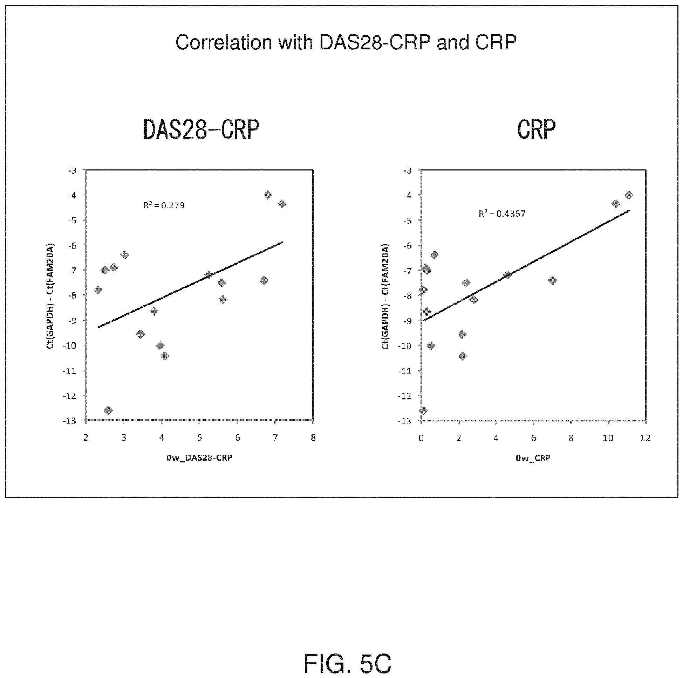

Fig. 5A shows the correlation between microarray data on *FAM20A* and PCR cycle number in all of the 27 samples. The correlation of data was very good (R² = 0.529). Fig. 5B shows the result of a comparative analysis about the amount of expression of *FAM20A* quantified by PCR between 12 cases of normal individuals and 15 cases of patients with rheumatoid arthritis. Expression accelerated significantly in patients with rheumatoid arthritis as compared with normal individuals. Furthermore, Fig. 5C analyzes the correlation between the amount of expression of *FAM20A* quantified by PCR and DAS28-CRP and CRP in the 15 cases of patients with rheumatoid arthritis. As in the case of the microarray data, a significant positive correlation was found between the expression of *FAM20A* and these clinical indicators.

The abovementioned results clearly show that both the "correlation between the expression of *FAM20A* in peripheral blood and the activity of rheumatoid arthritis in patients with rheumatoid arthritis" and the "difference in the expression of *FAM20A* in peripheral blood between normal individuals and patients with rheumatoid arthritis" are reproducible in the real-time PCR as well as in the microarray analysis.

## Claims

1. A method for determining a progression of rheumatoid arthritis in a subject or determining a therapeutic effect of a therapeutic drug for rheumatoid arthritis using a rheumatoid arthritis linked indicator in a subject, comprising:
measuring an amount of expression of the FAM20A gene in peripheral blood derived from the subject;
a) comparing the measured amount of expression of the FAM20A gene in the peripheral blood derived from the subject with a gene expression profile that is prepared based on an amount of expression of the FAM20A gene in peripheral blood collected from a patient with rheumatoid arthritis, when a progression of rheumatoid arthritis in a subject is determined ; or
b) comparing the measured amount of expression of the FAM20A gene in the peripheral blood derived from the subject after administration of a therapeutic drug with a gene expression profile that is prepared based on an amount of expression of the FAM20A gene in peripheral blood collected from the subject prior to administration of the therapeutic drug , when a therapeutic effect of a therapeutic drug for rheumatoid arthritis is determined, and wherein said gene expression profile shows a positive correlation between an amount of expression of the FAM20A gene and an indicator of a progression of rheumatoid arthritis in a subject;
estimating the value of the indicator in the subject on the basis of the comparing step; and
determining a progression of rheumatoid arthritis in a subject or determining a therapeutic effect of a therapeutic drug for rheumatoid arthritis from the estimated value of the indicator.

2. The method according to claim 1, wherein the indicator is selected from the group consisting of DAS28-CRP, DAS28-ESR, DAS44, EULAR improvement criteria, CDAI, SDAI, ACR and serum CRP concentration.

3. The method according to claim 1, wherein the indicator shows high activity of progression when the amount of expression of the FAM20A gene in peripheral blood derived from the subject is large.

4. The method according to claim 1, wherein the FAM20A gene consists of (i) a base sequence according to SEQ ID NO: 1 or (ii) a base sequence that hybridizes with the base sequence according to SEQ ID NO: 1 under stringent conditions, the base sequence being a base sequence encoding a protein having a function equivalent to that of a protein consisting of an amino acid sequence according to SEQ ID NO: 2.

5. The method according to claim 1, wherein the subject is suffering from rheumatoid arthritis.

6. The method according to claim 5, wherein the measuring measures amounts of expression of the FAM20A gene derived from peripheral blood before and after administering a therapeutic agent for rheumatoid arthritis to the subject, wherein a therapeutic effect of the therapeutic agent for rheumatoid arthritis is determined by analyzing the amounts of expression of the FAM20A gene in peripheral blood before and after administration using the gene expression profile.

## Patentansprüche

1. Ein Verfahren zur Bestimmung einer Progression rheumatoider Arthritis bei einem Probanden oder Bestimmung einer therapeutischen Wirkung eines therapeutischen Arzneimittels für rheumatoide Arthritis unter Verwendung eines mit rheumatoider Arthritis verknüpften Indikators bei einem Probanden,
umfassend:
Messen einer Expressionsmenge des Gens FAM20A in vom Probanden entnommenem peripherem Blut;
a) Vergleichen der gemessenen Expressionsmenge des Gens FAM20A in dem vom Probanden entnommenen peripheren Blut mit einem Genexpressionsprofil, welches auf der Grundlage einer Expressionsmenge des Gens FAM20A in von einem Probanden mit rheumatoider Arthritis abgenommenem peripherem Blut, wenn eine Progression rheumatoider Arthritis bei einem Probanden bestimmt wird; oder
b) Vergleichen der gemessenen Expressionsmenge des Gens FAM20A in dem vom Probanden nach Verabreichung eines therapeutischen Arzneimittels entnommenen peripheren Blut mit einem Genexpressionsprofil, welches angefertigt wird auf der Grundlage einer Expressionsmenge des Gens FAM20A in vom Probanden vor der Verabreichung des therapeutischen Arzneimittels abgenommenem peripherem Blut, wenn eine therapeutische Wirkung eines therapeutischen Arzneimittels für rheumatoide Arthrithis bestimmt wird,
und wobei besagtes Genexpressionsprofil eine positive Korrelation zwischen einer Expressionsmenge des Gens FAM20A und einem Indikator einer Progression rheumatoider Arthritis bei einem Probanden zeigt;
Schätzen des Werts des Indikators beim Probanden auf der Grundlage des Vergleichsschritts; und
Bestimmen einer Progression rheumatoider Arthritirs bei einem Probanden oder Bestimmen einer therapeutischen Wirkung eines therapeutischen Arzneimittels für rheumatoide Arthritis ausgehend vom geschätzten Wert des Indikators.

2. Das Verfahren gemäß Anspruch 1, wobei der Indikator ausgewählt wird aus einer Gruppe bestehend aus DAS28-CRP, DAS28-ESR, DAS44, EULAR-Verbesserungskriterien, CDAI, SDAI, ACR und Serum-CRP-Konzentration.

3. Das Verfahren gemäß Anspruch 1, wobei der Indikator eine hohe Progressionsaktivität zeigt, wenn die Expressionsmenge des Gens FAM20A in vom Probanden entnommenem peripherem Blut groß ist.

4. Das Verfahren gemäß Anspruch 1, wobei das Gen FAM20A besteht aus (i) einer Basissequenz gemäß SEQ ID NR.1 oder (ii) einer Basissequenz, welche unter strengen Bedingungen mit der Basissequenz gemäß SEQ ID Nr.1 hybridisiert, wobei die Basissequenz eine Basissequenz ist, welche ein Protein codiert, das eine Funktion aufweist, die der eines aus einer Aminosäuresequenz gemäß SEQ ID Nr. 2 bestehenden Proteins entspricht.

5. Das Verfahren gemäß Anspruch 1, wobei der Proband unter rheumatoider Arthritis leidet.

6. Das Verfahren gemäß Anspruch 5, wobei beim Messen Expressionsmengen des Gens FAM20A gemessen werden, welche von peripherem Blut vor und nach Verabreichung des therapeutischen Wirkstoffes für rheumatoide Arthritis an den Probanden stammen, wobei eine therapeutische Wirkung des therapeutischen Wirkstoffes für rheumatoide Arthritis durch Analysieren der Expressionsmengen des Gens FAM20A in peripherem Blut vor und nach Verabreichung unter Verwendung des Genexpressionsprofils ermittelt wird.

## Revendications

1. Procédé de détermination d'une progression d'arthrite rhumatoïde chez un sujet ou de détermination d'un effet thérapeutique d'un médicament thérapeutique pour l'arthrite rhumatoïde utilisant un indicateur lié à l'arthrite rhumatoïde chez un sujet, comprenant :
le mesurage d'une quantité d'expression du gène FAM20A dans du sang périphérique provenant du sujet ;
a) la comparaison de la quantité d'expression mesurée du gène FAM20A dans le sang périphérique provenant du sujet avec un profil d'expression génique qui est préparé sur la base d'une quantité d'expression du gène FAM20A dans du sang périphérique recueilli chez un patient atteint d'arthrite rhumatoïde, lorsque l'on détermine la progression de l'arthrite rhumatoïde chez un sujet ; ou
b) la comparaison de la quantité d'expression mesurée du gène FAM20A dans le sang périphérique provenant du sujet après l'administration d'un médicament thérapeutique avec un profil d'expression génique qui est préparé sur la base d'une quantité d'expression du gène FAM20A dans du sang périphérique recueilli chez le sujet avant l'administration du médicament thérapeutique, lorsque l'on détermine un effet thérapeutique d'un médicament thérapeutique pour l'arthrite rhumatoïde,
et dans lequel ledit profil d'expression génique présente une corrélation positive entre une quantité d'expression du gène FAM20A et un indicateur d'une progression de l'arthrite rhumatoïde chez un sujet ;
l'estimation de la valeur de l'indicateur chez le sujet sur la base de l'étape de comparaison ; et
la détermination de la progression de l'arthrite rhumatoïde chez un sujet ou la détermination d'un effet thérapeutique d'un médicament thérapeutique de l'arthrite rhumatoïde à partir de la valeur estimée de l'indicateur.

2. Procédé selon la revendication 1, dans lequel l'indicateur est choisi dans le groupe constitué de DAS28-CRP, DAS28-ESR, DAS44, critères d'amélioration EULAR, CDAI, SDAI, ACR et concentration sérique de CRP.

3. Procédé selon la revendication 1, dans lequel l'indicateur présente une activité élevée de progression lorsque la quantité d'expression du gène FAM20A dans du sang périphérique provenant du sujet est importante.

4. Procédé selon la revendication 1, dans lequel le gène FAM20A est constitué (i) d'une séquence de bases selon la SEQ ID NO : 1 ou (ii) d'une séquence de bases qui s'hybride avec la séquence de bases selon la SEQ ID NO : 1 dans des conditions rigoureuses, la séquence de bases étant une séquence de bases codant pour une protéine ayant une fonction équivalente à celle d'une protéine constituée d'une séquence d'acides aminés selon la SEQ ID NO : 2.

5. Procédé selon la revendication 1, le sujet étant atteint d'arthrite rhumatoïde.

6. Procédé selon la revendication 5, dans lequel le mesurage mesure des quantités d'expression du gène FAM20A obtenu à partir de sang périphérique avant et après l'administration d'un agent thérapeutique pour l'arthrite rhumatoïde au sujet, un effet thérapeutique de l'agent thérapeutique pour l'arthrite rhumatoïde étant déterminé en analysant les quantités d'expression du gène FAM20A dans le sang périphérique avant et après l'administration en utilisant le profil d'expression génique.


### SEQUENCE LISTING

<110> DNA Chip Research Inc.; KEIO UNIVERSIRY; SAITAMA MEDICAL UNIVERSITY
<120> METHOD FOR IDENTIFYING AN INDEX OF ACTIVITY OF RHEUMATOID ARTHRITIS
   AND BIOMARKER USED IN THE METHOD
<130> Y11S032PCT
<160> 6
<170> PatentIn version 3.1
<210> 1
   <211> 2267
   <212> DNA
   <213> Homo Sapiens
<400> 1
   aaaggcgcaa gaagcgggca ccccgggaac cccattccct cggctcactc ggcgcggaga      60
   agcgacgccc gctgactccg agagccccgg tgctccgtgc acctggtccc caagttgagg     120
   agcgacaccc ctccacaggg gactagcccg cgcggggagc attccggtct cactgacccc     180
   ggcccacccg cgggactcca ggcacctctt ctgcccgcac cccgcgaccc ctcccgggac     240
   cccggagaca gccggcctgc ccccggcgtc ccccttggcc agcacgccat gccggggctg     300
   cgccgggacc gcctactgac tctgctactg ctgggcgcgc tgctctccgc cgacctctac     360
   ttccacctct ggccccaagt acagcgccag ctgcggcctc gggagcgccc gcgggggtgc     420
   ccgtgcaccg gccgcgcctc ctccctggcg cgggactcgg ccgcagctgc ctcggacccc     480
   ggcacgatcg tgcacaactt ttcccgaacc gagccccgga ctgaaccggc tggcggcagc     540
   cacagcgggt cgagctccaa gttgcaggcc ctcttcgccc acccgctgta caacgtcccg     600
   gaggagccgc ctctcctggg agccgaggac tcgctcctgg ccagccagga ggcgctgcgg     660
   tattaccgga ggaaggtggc ccgctggaac aggcgacaca agatgtacag agagcagatg     720
   aaccttacct ccctggaccc cccactgcag ctccgactcg aggccagctg ggtccagttc     780
   cacctgggta ttaaccgcca tgggctctac tcccggtcca gccctgttgt cagcaaactt     840
   ctgcaagaca tgaggcactt tcccaccatc agtgctgatt acagtcaaga tgagaaagcc     900
   ttgctggggg catgtgactg cacccagatt gtgaaaccca gtggggtcca cctcaagctg     960
   gtgctgaggt tctcggattt cgggaaggcc atgttcaaac ccatgagaca gcagcgagat    1020
   gaagagacac cagtggactt cttctacttc attgactttc agagacacaa tgctgagatc    1080
   gcagctttcc atctggacag gattctggac ttccgacggg tgccgccaac agtggggagg    1140
   atagtaaatg tcaccaagga aatcctagag gtcaccaaga atgaaatcct gcagagtgtt    1200
   ttctttgtct ctccagcgag caacgtgtgc ttcttcgcca agtgtccata catgtgcaag    1260
   acggagtatg ctgtctgtgg caaaccacac ctgctggagg gttccctctc tgccttcctg    1320
   ccgtccctca acctggcccc caggctgtct gtgcccaacc cctggatccg ctcctacaca 1380
   ctggcaggaa aagaggagtg ggaggtcaat cccctttact gtgacacagt gaaacagatc 1440
   tacccgtaca acaacagcca gcggctcctc aatgtcatcg acatggccat cttcgacttc 1500
   ttgataggga atatggaccg gcaccattat gagatgttca ccaagttcgg ggatgatggg 1560
   ttccttattc accttgacaa cgccagaggg ttcggacgac actcccatga tgaaatctcc 1620
   atcctctcgc ctctctccca gtgctgcatg ataaaaaaga aaacactttt gcacctgcag 1680
   ctgctggccc aagctgacta cagactcagc gatgtgatgc gagaatcact gctggaagac 1740
   cagctcagcc ctgtcctcac tgaaccccac ctccttgccc tggatcgaag gctccaaacc 1800
   atcctaagga cagtggaggg gtgcatagtg gcccatggac agcagagtgt catagtcgac 1860
   ggcccagtgg aacagtcggc cccagactct ggccaggcta acttgacaag ctaagggctg 1920
   gcagagtcca gtttcagaaa atacgcctgg agccagagca gtcgactcga gtgccgaccc 1980
   tgcgtcctca ctcccacctg ttactgctgg gagtcaagtc agctaggaag gaagcaggac 2040
   attttctcaa acagcaagtg gggcccatgg aactgaatct ttactccttg gtgcaccgct 2100
   tctgtcgtgc gttgccttgc tccgtttttc ccaaaaagca ctggcttcat caaggccacc 2160
   gacgatttcc tgagtgcact gggaaatttg ggtataggtc aggcttggca gccttgatcc 2220
   caggagagta ctaatggtaa caagtcaaat aaaaggacat caagtgg 2267
<210> 2
   <211> 541
   <212> PRT
   <213> Homo Sapiens
<400> 2
<210> 3
   <211> 21
   <212> DNA
   <213> Homo Sapiens
<400> 3
   cgacactccc atgatgaaat c 21
<210> 4
   <211> 19
   <212> DNA
   <213> Homo Sapiens
<400> 4
   gctgcaggtg caaaagtgt 19
<210> 5
   <211> 19
   <212> DNA
   <213> Homo Sapiens
<400> 5
   agccacatcg ctcagacac 19
<210> 6
   <211> 19
   <212> DNA
   <213> Homo Sapiens
<400> 6
   gcccaatacg accaaatcc 19